# EUROPEAN PATENT SPECIFICATION

(11) **EP 1 546 135 B1**
(45) Date of publication and mention of the grant of the patent: **27.09.2006**
(21) Application number: 03764805.2
(22) Date of filing: 16.07.2003
(51) Int. Cl.: C07D 403/10

(54) **NOVEL SYNTHESIS OF IRBESARTAN**
NEUE SYNTHESE VON IRBESARTAN
NOUVEAU PROCEDE DE SYNTHESE D'IRBESARTAN

(30) Priority: 16.07.2002 US 396424 P; 09.08.2002 US 402490 P
(43) Date of publication of application: 29.06.2005
(73) Proprietor: TEVA PHARMACEUTICAL INDUSTRIES LTD., 49131 Petah Tiqva (IL)
(72) Inventor: NISNEVICH, Gennady, 34464 Haifa (IL); RUKHMAN, Igor, Technion City, Haifa 32000 (IL); PERTSIKOV, Boris, 36781 Nesher (IL); KAFTANOV, Julia, 35471 Haifa (IL); DOLITZKY, Ben-Zion, 49651 Petach Tiqva (IL)
(74) Representative: Gallagher, Kirk James
(86) International application number: PCT/US2003/022479
(87) International publication number: WO 2004/007482

(56) References cited:
- WO-A-99/38847
- US-A- 5 270 317
- LE BOURDONNEC, B.: "Synthesis and Pharmacological Evaluation of New Pyrazolidine-3,5-diones as AT1 Angiotensin II Receptor Antagonists" J. MED. CHEM., vol. 43, no. 14, 2000, pages 2685-2697, XP002259509

## Description

The present invention relates to a novel synthesis of irbesartan.

### BACKGROUND OF THE INVENTION

Irbesartan is a known angiotensin II receptor antagonist (blocker). Angiotensin is an important participant in the renin-angiotensin-aldosterone system (RAAS) and has a strong influence on blood pressure. The structure of irbesartan is shown below (I).

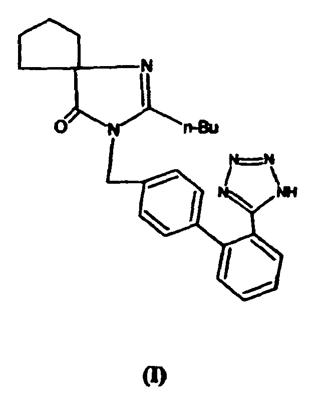

The synthesis of irbesartan is discussed, *inter alia*, in United States Patents 5,270,317 and 5,559,233. In the synthesis therein disclosed, the prepenultimate reaction step (exclusive of work-up and purification) involves the reaction of a cyano group on the biphenyl ring with an azide, for example tributyltin azide. Reaction time as long as 210 hours can be required. *See, e*.*g*., '317 patent.

United States Patent 5,629,331 also discloses a synthesis of irbesartan from a precursor 2-*n*-butyl-3-[(2'-cyanobiphenyl-4-yl)methyl]-1,3-diazaspiro[4.4]non-1-ene-4-one with sodium azide using a dipolar aprotic solvent. As acknowledged in the '331 patent, there are safety risks involved in the use of azides (column 4, line 39). Also, dipolar aprotic solvents (*e.g.* methyl pyrrolidone) are relatively high boiling and can be difficult to remove.

There is a need for an improved synthetic route to irbesartan.

### SUMMARY OF THE INVENTION

In one aspect, the present invention relates to a method of making irbesartan including the step of reacting 2-butyl-1,3-diazaspiro[4.4]non-1-ene-4-one and 5-(4'-bromomethylbiphenyl-2-yl)-1-trityl-1*H*-tetrazole in the presence of a phase transfer catalyst in a reaction system having first and second phases.

In another aspect, the present invention relates to a method of making irbesartan including the step of reacting 2-butyl-1,3-diazaspiro[4.4]non-1-ene-4-one and 5-(4'-bromomethylbiphenyl-2-yl)-1-trityl-1*H*-tetrazole in the presence of a phase transfer catalyst in a reaction system having first and second phases, wherein the first phase includes a first solvent that is an aromatic or aliphatic hydrocarbon and the second phase includes water and an inorganic base, for example KOH, NaOH, or LiOH, especially KOH.

In another aspect, the present invention relates to a method of making irbesartan including the step of reacting 2-butyl-1,3-diazaspiro[4.4]non-1-ene-4-one and 5-(4'-bromomethylbiphenyl-2-yl)-1-trityl-1*H*-tetrazole in the presence of a phase transfer catalyst that is a quaternary ammonium compound in a reaction system having first and second phases, wherein the first phase includes a first solvent that is an aromatic or aliphatic hydrocarbon and the second phase includes water and an inorganic base, for example KOH, NaOH, or LiOH, especially KOH.

In yet another aspect, the present invention relates to a method of making irbesartan including the steps of reacting 2-butyl-1,3-diazaspiro[4.4]non-1-ene-4-one and 5-(4'-bromomethylbiphenyl-2-yl)-1-trityl-1*H*-tetrazole in the presence of tetrabutylammonium hydrogensuflate in a reaction system having first and second phases, wherein the first phase includes a first solvent that is toluene and the second phase includes water and an inorganic base, especially KOH.

### BRIEF DESCRIPTION OF THE FIGURES

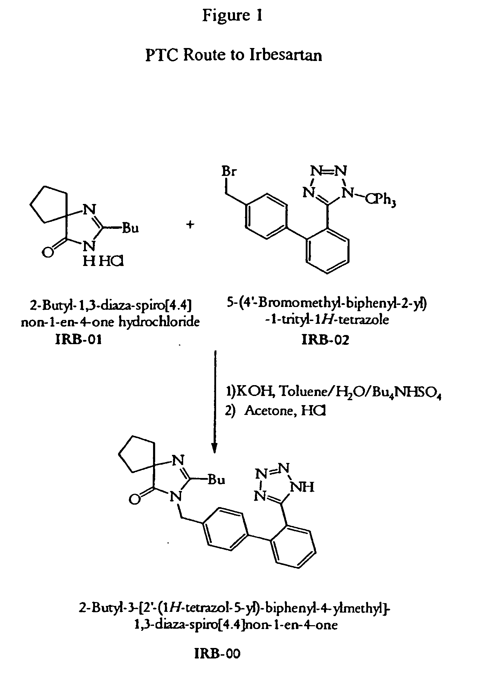

Figure 1 is schematic diagram of the process for making irbesartan of the present invention.

### DETAILED DESCRIPTION OF THE INVENTION

The present invention provides a novel synthesis of irbesartan in a two-phase reaction system having first and second liquid phases. The reaction is carried out in the presence of a phase transfer catalyst.

The first and second phases include first and second solvents, respectively, which are substantially immiscible in each other so that, when combined in a reaction vessel, a two-phase system is formed. Solvents are substantially immiscible in each other when equal volumes of them are mixed together, a two-phase system is formed in which the volume of the two phases is essentially equal. Preferably, substantially immiscible solvents are soluble in each other to the extent of about 1% (weight basis) or less.

First solvents can be aromatic or aliphatic hydrocarbons. Preferred first solvents are aromatic hydrocarbons. Examples of preferred aromatic hydrocarbons include benzene, toluene, m-xylene, o-xylene, and the tetralins, to mention just a few. Other aromatic hydrocarbons useful in the practice of the present invention will be apparent to the skilled artisan. Toluene is a particularly preferred aromatic hydrocarbon for use as first solvent.

The second solvent includes water. Water can be used alone or, preferably, an inorganic base such as KOH, NaOH or LiOH, to mention just a few, is combined with the water. The preferred inorganic base is KOH. Preferably, the water of the second phase contains a molar amount of base that is about 7 to about 12 times the molar amount of the diazaspiro or biphenyl reactants discussed below.

Phase transfer catalysts are well known to one skilled in the art of organic synthesis. Phase transfer catalysts are of particular utility when at least first and second compounds to be reacted with each other have such different solubility characteristics that there is no practical common solvent for them and, accordingly, combining a solvent for one of them with a solvent for the other of them results in a two-phase system.

Typically, when such compounds are to be reacted, the first reactant is dissolved in a first solvent and the second reactant is dissolved in a second solvent. Because the solvent for the first reactant is essentially insoluble in the solvent for the second reactant, a two-phase system is formed and reaction occurs at the interface between the two phases. The rate of such an interfacial reaction can be greatly increased by use of a phase transfer catalyst (PTC).

Several classes of compounds are known to be capable of acting as phase transfer catalysts, for example quaternary ammonium compounds and phosphonium compounds, to mention just two. Tetrabutylammonium hydrogensalfate is a preferred PTC for use in the practice of present invention.

In a first step of the synthetic method of the present invention, 2-butyl-3-[2'-(triphenylmethyltetrazol-5-yl)-biphenyl-4-yl methyl]-1,3-diazaspiro[4.4]non-1-ene-4-one (IRB-03) is obtained. In this step, a first solution of 5-(4'-bromomethylbiphenyl-2-yl)-1-trityl-1H-tetrazole (IRB-02) in a first solvent is provided. IRB-02 is known in the art and is disclosed, for example, in United States Patent 5,128,355.

Also to be provided is a second solution that includes 2-butyl-1,3-diazaspiro[4,4]non-1-ene-4-one (IRB-01), water, PTC, and a base, preferably an inorganic base, most preferably, KOH. The base is present in an amount between about 7 and about 12 molar equivalents relative to the number of moles of IRB-01. 2-Butyl-1,3-diazaspiro[4.4]non-1-ene-4-one is known in the art and is disclosed, for example, in United States Patent 5,559,233.

The first and second solutions, and their constituents, are combined in any order to form a two-phase reaction system that has first and second phases. The combining can be in any suitable vessel that is equipped with means for vigorous agitation of the reaction system to maximize the interfacial area between the two phases. The combining can be at any temperature from about 20° C to about 95° C, preferably at about 90°C. The reaction is allowed to proceed in the two phase system for a time that the skilled artisan will known to adjust according to the reaction temperature. When the reaction temperature is about 90° C, a reaction time between about 1 and about 2 hours is usually sufficient.

After the reaction time and to facilitate phase separation, the reaction system is allowed to cool, preferably to a temperature of about 15°C to about 30°C and the first (organic, aromatic hydrocarbon) and second (aqueous) phases are separated. If desired, the aqueous phase can be extracted one or more times with toluene and the extract(s) combined with the first (organic, aromatic hydrocarbon) phase. Solvent is removed from the separated first phase, preferably by evaporation, especially at reduced pressure, to afford a crude residue.

In a second step of the synthetic method of the present invention, the trityl group is cleaved from the tetrazole ring. Crude residue is dissolved in a suitable water-miscible solvent. A solvent is water miscible if it is miscible with water at least in any proportion from 80:20 to 20:80 (weight basis). Acetone is a preferred water-miscible solvent. The resulting solution is acidified, preferably with a mineral or sulfuric acid, and agitated at a temperature between about 15°C and about 30°C. The time of the cleavage reaction can be conveniently monitored using thin layer chromatography. The acid is neutralized (that is, the solution is basified) with a molar excess of base, preferably and inorganic base, most preferably aqueous KOH. The basification is to a pH of about 8 to about 12, preferably to a pH of about 9 to about 10.5. Water-miscible solvent is evaporated, preferably at reduced pressure, to concentrate the basified solution whereby a suspension id formed. The order of basification and evaporation is not important. That is, water-miscible solvent can be first evaporated, followed by basification of the concentrate.

The trityl alcohol formed is separated and the liquid phase is acidified (e.g. to a pH of about 2 to about 3.5), preferably with mineral acid, most preferably with HCl. The resulting suspension is cooled and the product recovered by, for example, filtration. If desired, the isolated product can be washed with an organic solvent, preferably a lower aliphatic alcohol, most preferably *iso*-propanol, and dried, preferably at reduced pressure.

In another embodiment, the present invention provides fine particle size or "micronized" irbesartan in cluding a plurality of irbesartan particles wherein the mean particle size (d_{.05}) is about 2 µm to about 7 µm and 10 volume percent or less of the plurality of particles have a particle diameter equal to or greater than about 30 µm, preferably 20 µm.

Micronized irbesartan including a plurality of irbesartan particles can be obtained by comminution using a fluid energy mill, wherein the mean particle size (d_{.05}) produced is about 2 µm to about 7 µm and 10 volume percent or less of the plurality of particles have a particle diameter equal to or greater than about 10 µm.

A fluid energy mill, or "micronizer", is an especially preferred type of mill for its ability to produce particles of small size in a narrow size distribution, i.e., micronized material. As those skilled in the art are aware, micronizers use the kinetic energy of collision between particles suspended in a rapidly moving fluid (typically air) stream to cleave the particles. An air jet mill is a preferred fluid energy mill. The suspended particles are injected under pressure into a recirculating particle stream. Smaller particles are carried aloft inside the mill and swept into a vent connected to a particle size classifier such as a cyclone. The feedstock should first be milled to about 150 to 850 µm which may be done using a conventional ball, roller, or hammer mill.

The starting material may have an average particle size of about 20-100 microns.

The material is fed into the micronization system in a controlled feed rate by means of a screw feeder or a vibratory feeder. The air jet mill is operated with controlled air pressures. For the Microgrinding MC-500 KX, the feed rate is 40-80 kg/hr, the Feed air pressure is 6-8.5 bar and the grinding air is 3-6 bar.

Micronizationization can also be accomplished with a pin mill. The starting material may have an average particle size of about 20-100 microns. The material is fed into the mill system in a controlled feed rate by means of a screw feeder or a vibratory feeder. The mill is operated with controlled speed. For the Alpine UPZ 160, the feed rate is 60-75 kg/hr, the mill speed is 7,000-15,000 rpm.

Micronized irbesartan can be used to make pharmaceutical compositions that can be in the form of solid oral dosage forms, for example compressed tablets. Compressed tablets can be made by dry or wet granulation methods as is known in the art. In addition to the pharmaceutically active agent or drug, compressed tablets contain a number of pharmacologically inert ingredients, referred to as excipients. Some excipients allow or facilitate the processing of the drug into tablet dosage forms. Other excipients contribute to proper delivery of the drug by, for example, facilitating disintegration.

The present invention can be illustrated in one of its embodiments by the following non-limiting example.

### Examples

### Example 1:

A solution of KOH (10.4 g, 157.0 mmol), IRB-01 (12.0 g, 52.0 mmol) and Bu₄NHSO₄ (1.8g, 5.3 mmol) in water (40 mL) was added to a solution of IRB-02 (24.6 g, 44.1 mmol) in toluene (240 mL), and the resulting two-phase mixture was heated at 90°C with vigorous stirring for 1.5 hours. The mixture was cooled to room temperature, the phases were separated, and the aqueous phase was extracted with toluene (50mL). The combined organics were evaporated; the residue was dissolved in acetone (100 mL) and 3N HC1 (52 mL, 156 mmol, 3 eq) and stirred at room temperature (TLC monitoring). A solution of KOH (14.6 g, 260 mmol, 5 eq) in water (100 mL) was slowly added, and acetone was evaporated under reduced pressure. The precipitate formed (trityl alcohol) was filtered and washed with water (2 x 50 mL); the filtrate was washed with toluene and slowly acidified to pH 4 with 3N HCl. The resulting suspension was cooled to 0-4°C, stirred for additional 30 min and filtered. The cake was washed with cold *iso*-propanol (2 x 25 mL) and dried under reduced pressure at 50-60°C; affording crude IRB-00 (14.5g, 33.8 mmol). Yield 84.3%, purity 94% (by HPLC).

### Example 2:

A solution of H₂SO₄ (98 %, 22.6 g, 12.3 mL, 0.225 mol, 1.5 eq) in water (160 mL) was added to a suspension of IRB-03 (100.6 g, 0.150 mol) in acetone (600 mL) at 35-40 °C and stirred for 7 h (suspension disappeared; TLC monitoring ― Hexane / EtOAc = 1:1). Acetone was evaporated from the reaction mixture under reduced pressure at 30-40 °C.

Water (500 mL) was added to the resulting suspension. The resulting mixture was vigorously stirred and cooled to 0-5 °C. A solution of KOH (85 %, 39.6 g, 0.600 mol, 4 eq) in water (100 mL) was slowly added keeping the reaction temperature below 15 °C and the mixture was stirred for 30 min until a stable pH (9-10) was obtained. Then, a second portion of KOH (3.0 g, 50 mmol, 0.3 eq) in water (10 mL) was added and the reaction was stirred for additional 30 min at 5-10 °C (pH 10.5-11.5). The precipitate (triphenyl methanol) was filtered, washed with water (2 x 100 mL) and dried under reduced pressure (10 mmHg) at 50 °C to give 36.5 g (about 95 % yield) of triphenyl methanol. The aqueous filtrate was extracted with ethyl acetate (300 mL), cooled to 10 °C and acidified to pH 2.0-3.5 with slow addition of 20 % aqueous H₂SO₄. The resulting suspension was stirred at 0-4 °C for an additional 30 min and filtered. The filter cake was washed twice with water (2 x 100 mL), then with EtOAc (100 mL) and dried under reduced pressure for 3 h at 50 °C afforded 60.0 g (93 % yield) of crude Irbesartan. The crude product (60.0 g) was refluxed in 95 % aqueous ethanol (600 mL) for 1 h (clear solution was formed) and allowed to cool to room temperature with vigorous stirring. The mixture was stirred for an additional 2 h at 0-5 °C, filtered, and washed with cold 95 % aqueous ethanol (100 mL). The collected solid was dried under reduced pressure (3 h, 50 °C, 10 mmHg) afforded 56.0 g (93 % yield), of a white powder.

## Claims

1. A method of making 2-butyl-3-[2'-(triphenylmethyl tetrazol-5-yl)-biphenyl-4-yl methyl]-1,3-diazaspiro[4.4]non-1-ene-4-one comprising the step of reacting 2-butyl-1,3-diazaspiro[4.4]non-1-ene-4-one and 5-(4'-bromomethylbiphenyl-2-yl)-1-trityl-1*H*-tetrazole in the presence of a phase transfer catalyst in a reaction system comprising first and second phases.

2. The method of claim 1 wherein the first phase comprises an aromatic or aliphatic hydrocarbon and the second phase comprises water.

3. The method of claim 1 or claim 2 wherein, prior to reaction, the 2-butyl-1,3-diazaspiro[4.4]non-1-ene-4-one is in solution in aqueous base.

4. The method of claim 3 wherein the aqueous base is selected from the group consisting of KOH, NaOH and LiOH.

5. The method of claim 4 wherein the aqueous base is aqueous KOH.

6. The method of any of claims 2 to 5 wherein, prior to reaction, the 5-(4'-bromomethylbiphenyl-2-yl)-1-trityl-1*H*-tetrazole is in solution in an aromatic or aliphatic hydrocarbon.

7. The method of claim 6 wherein the 5-(4'-bromomethylbiphenyl-2-yl)-1-trityl-1*H*-tetrazole is in solution in an aromatic hydrocarbon that is toluene.

8. The method of claim 5 wherein the 5-(4'-bromomethylbiphenyl-2-yl)-1-trityl-1*H-*tetrazole is in solution in an aliphatic hydrocarbon.

9. The method of any preceding claim wherein the phase transfer catalyst is a quaternary ammonium compound.

10. The method of claim 9 wherein the quaternary ammonium compound is tetrabutyl ammonium hydrogensulfate.

11. A method for making irbesartan which comprises preparing 2-butyl-3-[2'-(triphenylmethyltetrazol-5-yl)-biphenyl-4-yl methyl]-1,3-diazaspiro[4.4]non-1-ene-4-one prepared according to any preceding claim and further cleaving the trityl group from the tetrazole ring.

12. A method for making irbesartan comprising the steps of preparing 2-butyl-3-[2'-(triphenylmethyltetrazol-5-yl)-biphenyl-4-yl methyl]-1,3-diazaspiro[4.4]non-1-ene-4-one prepared according to the method of any one of claims 1 to 10; heating the combination to a temperature of about 20°C and about 95°C; separating the first and second phases; removing solvent from the first phase to obtain a residue; providing a mineral acid acidified solution of the residue in a water-miscible solvent, basifying the solution in water-miscible solvent with an inorganic base; removing water-miscible solvent from the solution; separating trityl alcohol so formed; and recovering irbesartan.

13. The method of claim 12 wherein the water miscible solvent is acetone.

14. The method of claim 12 wherein the basification is with an inorganic base to a pH of 8 to 12, preferably to a pH of 9 to 10.5.

## Patentansprüche

1. Verfahren zum Herstellen von 2-Butyl-3-[2'-(triphenylmethyltetrazol-5-yl)-biphenyl-4-yl-methyl]-1,3-diazaspiro[4.4]non-1-en-4-on, welches die Stufe umfaßt, bei der man 2-Butyl-1,3-diazaspiro[4.4]non-1-en-4-on und 5-(4'-Brommethylbiphenyl-2-yl)-1-trityl-1*H*-tetrazol in der Gegenwart eines Phasenübergangskatalysators in einem Reaktionssystem, welches erste und zweite Phasen umfaßt, umsetzt.

2. Verfahren nach Anspruch 1, wobei die erste Phase einen aromatischen oder aliphatischen Kohlenwasserstoff umfaßt und die zweite Phase Wasser umfaßt.

3. Verfahren nach Anspruch 1 oder Anspruch 2, wobei vor der Umsetzung das 2-Butyl-1,3-diazaspiro[4.4]non-1-en-4-on in Lösung in wäßriger Base vorliegt.

4. Verfahren nach Anspruch 3, wobei die wäßrige Base aus der Gruppe ausgewählt ist, bestehend aus KOH, NaOH und LiOH.

5. Verfahren nach Anspruch 4, wobei die wäßrige Base wäßriges KOH ist.

6. Verfahren nach einem der Ansprüche 2 bis 5, wobei vor der Umsetzung das 5-(4'-Brommethylbiphenyl-2-yl)-1-trityl-1*H*-tetrazol in Lösung in einem aromatischen oder aliphatischen Kohlenwasserstoff vorliegt.

7. Verfahren nach Anspruch 6, wobei das 5-(4'-Brommethylbiphenyl-2-yl)-1-trityl-1*H*-tetrazol in Lösung in einem aromatischen Kohlenwasserstoff, der Toluen ist, vorliegt.

8. Verfahren nach Anspruch 5, wobei das 5-(4'-Brommethylbiphenyl-2-yl)-1-trityl-1*H*-tetrazol in Lösung in einem aliphatischen Kohlenwasserstoff vorliegt.

9. Verfahren nach einem der vorangegangenen Ansprüche, wobei der Phasenübergangskatalysator eine quaternäre Ammoniumverbindung ist.

10. Verfahren nach Anspruch 9, wobei die quaternäre Ammoniumverbindung Tetrabutylammoniumhydrogensulfat ist.

11. Verfahren zum Herstellen von Irbesartan, welches das Herstellen von 2-Butyl-3-[2'-(triphenylmethyltetrazol-5-yl)-biphenyl-4-yl-methyl]-1,3-diazaspiro[4.4]non-1-en-4-on, hergestellt nach einem der vorangegangenen Ansprüche, und weiterhin das Abspalten der Tritylgruppe von dem Tetrazolring umfaßt.

12. Verfahren zum Herstellen von Irbesartan, welches die Stufen umfaßt, bei denen man 2-Butyl-3-[2'-(triphenylmethyltetrazol-5-yl)-biphenyl-4-yl-methyl]-1,3-diazaspiro[4.4]non-1-en-4-on, hergestellt nach dem Verfahren gemäß einem der Ansprüche 1 bis 10, herstellt, die Kombination auf eine Temperatur von zwischen etwa 20°C und etwa 95°C erwärmt, die erste und die zweite Phase trennt, unter Erhalt eines Rückstands Lösungsmittel aus der ersten Phase entfernt, eine mit Mineralsäure angesäuerte Lösung des Rückstands in einem mit Wasser mischbaren Lösungsmittel bereitstellt, die Lösung in einem mit Wasser mischbaren Lösungsmittel mit einer anorganischen Base alkalisiert, das mit Wasser mischbare Lösungsmittel aus der Lösung entfernt, so gebildeten Tritylalkohol abtrennt und Irbesartan gewinnt.

13. Verfahren nach Anspruch 12, wobei das mit Wasser mischbare Lösungsmittel Aceton ist.

14. Verfahren nach Anspruch 12, wobei das Alkalisieren mit einer anorganischen Base auf einen pH-Wert von 8 bis 12, vorzugsweise auf einen pH-Wert von 9 bis 10,5, erfolgt.

## Revendications

1. Un procédé de fabrication de 2-butyl-3-[2'-(triphénylméthyltétrazol-5-yl)-biphényl-4-yl méthyl] 1,3-diazaspiro[4,4]non-1-ène-4-one comprenant l'étape de réaction de 2-butyl-1,3-diazaspiro[4,4]non-1-ène-4-one et de 5-(4'-bromo-méthyl-biphényl-2-yl)-1-trityl-1*H*-tétrazole en présence d'un catalyseur de transfert de phase dans un système réactionnel comprenant une première et une deuxième phases.

2. Le procédé selon la revendication 1, dans lequel la première phase comprend un hydrocarbure aromatique ou aliphatique et la deuxième phase comprend de l'eau.

3. Le procédé selon la revendication 1 ou la revendication 2, dans lequel, préalablement à la réaction, le 2-butyl-1,3-diazaspiro[4,4]non-1-ène-4-one est en solution dans une base aqueuse.

4. Le procédé selon la revendication 3, dans lequel la base aqueuse est choisie dans le groupe comprenant KOH, NaOH et LiOH..

5. Le procédé selon la revendication 4, dans lequel la base aqueuse consiste en KOH en solution aqueuse.

6. Le procédé selon une quelconque des revendications 2 à 5, dans lequel, préalablement à la réaction, le 5-(4'-bromométhylbiphényl-2-yl)-1-trityl-1*H-*tétrazole est en solution dans un hydrocarbure aromatique ou aliphatique.

7. Le procédé selon la revendication 6, dans lequel le 5-(4'-bromo-méthylbiphényl-2-yl)-1-trityl-1*H*-tétrazole est en solution dans un hydro-carbure aromatique consistant en toluène.

8. Le procédé selon la revendication 5, dans lequel le 5-(4'-bromo-méthylbiphényl-2-yl)-1-trityl-1*H*-tétrazole est en solution dans un hydro-carbure aliphatique.

9. Le procédé selon une quelconque des revendications précédentes, dans lequel le catalyseur de transfert de phase est un dérivé d'ammonium quaternaire.

10. Le procédé la revendication 9, dans lequel le dérivé d'ammonium quaternaire est un hydrogénosulfate de tétrabutylammonium..

11. Un procédé de fabrication d'irbesartan qui comprend la préparation de 2-butyl-3-[2'-(triphénylméthyltétrazol-5-yl)-biphényl-4-yl méthyl] 1,3-diazaspiro-[4,4]non-1-ène-4-one préparé selon une quelconque des revendications précédentes, et en outre la séparation du groupe trityle du noyau tétrazole.

12. Un procédé de fabrication d'irbesartan qui comprend les étapes de préparation de 2-butyl-3-[2'-(triphénylméthyltétrazol-5-yl)-biphényl-4-yl méthyl] 1,3-diazaspiro-[4,4]non-1-ène-4-one préparé à l'aide du procédé selon une quelconque des revendications 1 à 10; de chauffage de l'ensemble à une température comprise entre environ 20°C et environ 95°C; de séparation des première et seconde phases; d'élimination du solvant contenu dans la première phase pour obtenir un résidu; d'obtention d'une solution, rendue acide par l'ajout d'un acide minéral, du résidu dans un solvant miscible à l'eau; de basification de la solution dans le solvant miscible à l'eau par addition d'une base inorganique; d'élimination du solvant miscible à l'eau contenu dans ladite solution; de séparation de l'alcool tritylique ainsi formé et de récupération de l'irbesartan.

13. Le procédé selon la revendication 12, dans lequel le solvant miscible à l'eau consiste en acétone.

14. Le procédé selon la revendication 12, dans lequel la basification est réalisée à l'aide d'une base inorganique à un pH de 8 à 12, de préférence de 9 à 10,5
